(19) Europäisches Patentamt
European Patent Office
Office européen des brevets

(11) EP 3 639 826 A1

(12) # EUROPEAN PATENT APPLICATION
published in accordance with Art. 153(4) EPC

(43) Date of publication:
**22.04.2020 Bulletin 2020/17**

(21) Application number: **18818391.7**

(22) Date of filing: **15.06.2018**

(51) Int Cl.:
***A61K 31/4709*** *(2006.01)* ***A61P 11/00*** *(2006.01)*
***A61P 31/04*** *(2006.01)*

(86) International application number:
**PCT/JP2018/022846**

(87) International publication number:
**WO 2018/230686 (20.12.2018 Gazette 2018/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.06.2017 US 201762520961 P**
**30.03.2018 JP 2018068159**

(71) Applicant: **Kyorin Pharmaceutical Co., Ltd.**
**Tokyo 101-8311 (JP)**

(72) Inventors:
- **ODAJIMA, Masaaki**
**Tokyo 101-8311 (JP)**
- **TANIOKA, Sayoko**
**Tokyo 101-8311 (JP)**
- **SUNOUCHI, Takaaki**
**Tokyo 101-8311 (JP)**
- **TABUCHI, Asako**
**Tokyo 101-8311 (JP)**

(74) Representative: **Vossius & Partner**
**Patentanwälte Rechtsanwälte mbB**
**Siebertstrasse 3**
**81675 München (DE)**

(54) **THERAPEUTIC AGENT FOR ASPIRATION PNEUMONIA, LUNG SUPPURATION, OR LUNG ABSCESS**

(57) [Problem]
The present invention pertains to a safer and more efficient therapeutic agent for respiratory tract infections.
[Solution]
A therapeutic agent for aspiration pneumonia, lung suppuration, or lung abscess, the agent containing as an active ingredient 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof.

EP 3 639 826 A1

## Description

Technical Field

The present invention relates to a therapeutic agent for aspiration pneumonia, lung suppuration or lung abscess.

Background Art

Since the development of norfloxacin, quinolone carboxylic acid antibacterial agents called new quinolones have been developed all over the world, and many new quinolone antibacterial agents are now widely used as therapeutic drugs for infectious diseases.

Meanwhile, a quinolone carboxylic acid derivative represented by the general formula (1) has been disclosed by the applicant (Patent Literature 1).

[Formula 1]

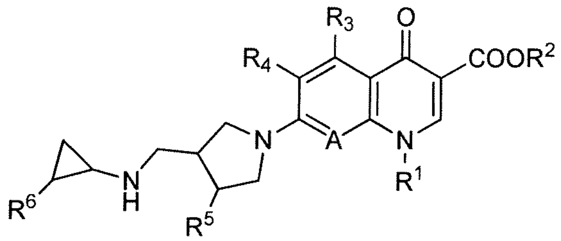

(I)

In formula (1), $R^1$ represents an alkyl group having 1 to 6 carbon atoms optionally substituted with one or more halogen atoms, a cycloalkyl group having 3 to 6 carbon atoms optionally substituted with one or more halogen atoms, or an aryl group or heteroaryl group optionally substituted with one or more same or different substituents selected from a halogen atom and an amino group, $R^2$ represents a hydrogen atom, an alkyl group having 1 to 3 carbon atoms, or a pharmaceutically acceptable cation, $R^3$ represents a hydrogen atom, a halogen atom, a hydroxyl group, an amino group or an alkyl group having 1 to 3 carbon atoms, $R^4$ represents a hydrogen atom or a halogen atom, $R^5$ represents a fluorine atom, $R^6$ represents a hydrogen atom or a fluorine atom, and A represents a nitrogen atom or =C-X (wherein X represents a hydrogen atom, a halogen atom, an amino group, a cyano group, or an alkyl group having 1 to 3 carbon atoms or alkoxy group having 1 to 3 carbon atoms optionally substituted with one or more halogen atoms).

In addition, Patent Literature 1 discloses 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid as one of the quinolone carboxylic acid derivatives described above. Moreover, a hydrochloride salt thereof is disclosed in Patent Literature 2.

In addition, one example of respiratory infection includes aspiration pneumonia. Aspiration pneumonia is a disease that accounts for the majority of pneumonia in the elderly and is a serious disease that is refractory, relapsing, and has a high fatality rate (Non Patent Literature 1). The causative bacteria of aspiration pneumonia include anaerobic bacteria, Staphylococcus aureus, and enterobacteria (Non Patent Literature 1), but a method for effectively treating aspiration pneumonia has not been established so far. The quinolone preparations currently on the market include levofloxacin, ciprofloxacin, pazufloxacin, moxifloxacin, sitafloxacin and garenoxacin. For aspiration pneumonia, which is a disease of high severity, most of the initial treatments use injectable preparations, but among the quinolone preparations mentioned above, levofloxacin, ciprofloxacin and pazufloxacin, for which injectable preparations are available, have insufficient antibacterial activity against anaerobic bacteria and are not recommended for use in patients suspected of aspiration pneumonia (Non Patent Literature 2). In oral preparations, sitafloxacin, moxifloxacin and galenoxacin may be effective against anaerobic bacterial infections (Non Patent Literatures 3 to 5), but there are no reports of articles with high evidence for aspiration pneumonia, and no effective treatment method has been established so far.

An example of a respiratory infection mainly caused by anaerobic bacteria, as in the case of aspiration pneumonia, include lung abscess (Non Patent Literature 6). Although there are reports that therapeutic efficacy is observed for moxifloxacin and pazufloxacin (Non Patent Literatures 3 to 4 and Non Patent Literature 7), they have not been established as an effective treatment method so far.

Citation List

Non Patent Literature

Non Patent Literature 1: The Journal of the Japanese Society of Internal Medicine, 99:11, November 10, 2010, p. 2746-2751.
Non Patent Literature 2: The Japanese Respiratory Society, Medical/Care-related Pneumonia Clinical Practice Guidelines, p. 23.
Non Patent Literature 3: Infection (Munich, Germany) (2008), 36(1), 23-30.
Non Patent Literature 4: Expert Review of Respiratory Medicine (2007), 1(1), 111-119.
Non Patent Literature 5: The Japanese Respiratory Society, Adult Pneumonia Clinical Practice Guidelines 2017, p. 24.
Non Patent Literature 6: Annals of The Japanese Respiratory Society, 49(9): 623-628, 2011.
Non Patent Literature 7: Nippon Kagaku Ryoho Gakkai Zasshi (1999), 47(Suppl. 1), 196-203.

Patent Literature

Patent Literature 1: International Publication No. WO 2005/026147 pamphlet
Patent Literature 2: International Publication No. WO 2013/069297

Summary of Invention

Technical Problem

An object of the present invention is to provide a novel therapeutic agent for respiratory infections.

Solution to Problem

The present inventor has studied a therapeutic agent for respiratory infections that is highly effective and safe. The present inventors have made extensive studies on the above-mentioned problems, and found that 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid is extremely effective as a therapeutic agent for aspiration pneumonia, lung suppuration or lung abscess, and completed the present invention.
The gist of the present invention is as follows.

[1] A therapeutic agent for aspiration pneumonia, lung suppuration or lung abscess comprising 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient.
[2] A therapeutic agent for aspiration pneumonia comprising 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient.
[3] A therapeutic agent for lung suppuration or lung abscess comprising 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient.
[4] The therapeutic agent according to [1], wherein causative bacteria of the aspiration pneumonia, lung suppuration or lung abscess are one or more bacteria selected from the group consisting of bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia* and bacteria belonging to the genus *Fusobacterium.*
[5] The therapeutic agent according to [2], wherein causative bacteria of the aspiration pneumonia are one or more bacteria selected from the group consisting of bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia* and bacteria belonging to the genus *Fusobacterium.*
[6] The therapeutic agent according to [3], wherein causative bacteria of the lung suppuration or lung abscess are

one or more bacteria selected from the group consisting of bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia* and bacteria belonging to the genus *Fusobacterium.*

[7] The therapeutic agent according to [1], wherein causative bacteria of the aspiration pneumonia, lung suppuration or lung abscess are one or more bacteria selected from the group consisting of bacteria belonging to the genus *Bacteroides,* bacteria belonging to the genus *Prevotella,* bacteria belonging to the genus *Porphyromonas,* bacteria belonging to the genus *Fusobacterium,* bacteria belonging to the genus *Leptotrichia,* bacteria belonging to the genus *Peptostreptococcus,* bacteria belonging to the genus *Parvimonas,* bacteria belonging to the genus *Veillonella,* bacteria belonging to the genus Tissierella, *Streptococcus anginosus* group, and bacteria belonging to the genus *Actinomyces.*

[8] The therapeutic agent according to [2], wherein causative bacteria of the aspiration pneumonia are one or more bacteria selected from the group consisting of bacteria belonging to the genus *Bacteroides,* bacteria belonging to the genus *Prevotella,* bacteria belonging to the genus *Parvimonas,* bacteria belonging to the genus *Veillonella* and bacteria belonging to the genus *Actinomyces.*

[9] The therapeutic agent according to [3], wherein causative bacteria of the lung suppuration or lung abscess are one or more bacteria selected from the group consisting of bacteria belonging to the genus *Bacteroides,* bacteria belonging to the genus *Prevotella,* bacteria belonging to the genus *Porphyromonas,* bacteria belonging to the genus *Fusobacterium,* bacteria belonging to the genus *Leptotrichia,* bacteria belonging to the genus *Peptostreptococcus,* bacteria belonging to the genus Parvimonas, bacteria belonging to the genus *Veillonella,* bacteria belonging to the genus *Tissierella,* and *Streptococcus anginosus* group.

[10] The therapeutic agent according to [1], wherein a daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid.

[11] The therapeutic agent according to [2], wherein a daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid.

[12] The therapeutic agent according to [3], wherein a daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid.

## Advantageous Effects of Invention

According to the present invention, a therapeutic agent for aspiration pneumonia, lung suppuration or lung abscess, comprising administering 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof to a patient, can be provided.

## Description of Embodiment

The following describes in detail one embodiment of the present invention.

The therapeutic agent of the present embodiment relates to a therapeutic agent for respiratory diseases, and particularly relates to a therapeutic agent for respiratory infections. More specifically, the therapeutic agent of the present embodiment relates to a therapeutic agent for aspiration pneumonia, lung suppuration or lung abscess, comprising administering 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof to a patient, including a human.

A respiratory infection refers to an infection that occurs at any site in the respiratory tract. Moreover, respiratory tract is a general term for the organs related to respiration, and refers to organs from the nasal vestibule to the alveoli via the nasal cavity, pharynx, larynx, trachea, bronchi, and bronchioles.

"Aspiration pneumonia" herein is a respiratory condition including swelling and infection of the lungs and airways, and is thought to be caused by inhaling harmful substances. Patients with aspiration pneumonia may have symptoms such as coughing and dyspnea. A patient with aspiration pneumonia herein means a person who satisfies the following criteria.

- A clear infiltrative shadow appearing acutely on chest X-rays or CT images is observed.
- A clear aspiration has been confirmed, repetitive choking has been confirmed, dysfunction in the swallowing function evaluation test has been confirmed, or the patient has a complication or history of a disease with potential dysphagia.
- The patient presents with symptoms and inflammation that are characteristic of aspiration pneumonia.
  Cough, purulent sputum, wet rales, dyspnea, fever, CRP positive, leukocytosis, hypoxemia and so on are exemplified as the symptoms and inflammation that are characteristic of aspiration pneumonia.

"Lung suppuration" herein is a necrotizing pulmonary infection, which is also called a lung abscess and is thought to be caused by the inhalation of bacteria in the mouth and throat into the lungs. Patients with lung suppuration may have symptoms such as fatigue, loss of appetite, night sweats, fever, weight loss, and cough with sputum.
A patient with lung suppuration herein means a person who satisfies the following criteria.

- On chest X-rays or CT images, a massive shadow or a shadow with cavities inside (nodular shadow, mass shadow) is observed. (Regardless of the presence of a niveau due to pus accumulation.)
- The patient presents with symptoms and inflammation that are characteristic of lung suppuration/lung abscess. Cough, purulent sputum, wet rales, dyspnea, fever, CRP positive, leukocytosis, hypoxemia and so on are exemplified as the symptoms and inflammation that are characteristic of lung suppuration or lung abscess.

Finding a safe and effective compound against anaerobic pathogens is important to effectively treat diseases such as aspiration pneumonia, lung suppuration or lung abscess. The applicant has found that 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid and pharmaceutically acceptable salts thereof are effective against anaerobic pathogens, unlike other quinolone compounds. For example, an injection of a quinolone compound such as levofloxacin, ciprofloxacin or pazufloxacin is considered not suitable as a therapeutic agent for aspiration pneumonia (Non Patent Literature 2).
However, the applicant has found that 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid and pharmaceutically acceptable salts thereof are effective against anaerobic pathogens and effective in the treatment of aspiration pneumonia.
7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or pharmaceutically acceptable salts thereof can be produced, for example, according to the methods described in Patent Literature 1 or 2.
Obligate anaerobes that are causative bacteria of aspiration pneumonia, lung suppuration or lung abscess include bacteria belonging to the genus *Bacteroides,* bacteria belonging to the genus *Prevotella,* bacteria belonging to the genus *Porphyromonas,* bacteria belonging to the genus *Fusobacterium,* bacteria belonging to the genus *Leptotrichia,* bacteria belonging to the genus *Peptostreptococcus,* bacteria belonging to the genus *Parvimonas,* bacteria belonging to the genus *Veillonella,* bacteria belonging to the genus *Tissierella,* bacteria belonging to the genus *Peptoniphilus* and bacteria belonging to the genus *Finegoldia,* and facultative anaerobes include the *Streptococcus anginosus* group, which is included in the genus *Streptococcus,* and bacteria belonging to the genus *Actinomyces*. 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid exhibits a high antibacterial activity against the anaerobes described above and exhibits a high therapeutic effect against aspiration pneumonia, lung suppuration or lung abscess.
Examples of causative bacteria of aspiration pneumonia include bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia,* bacteria belonging to the genus *Fusobacterium,* bacteria belonging to the genus *Bacteroides* and bacteria belonging to the genus *Streptococcus.*
Regarding the treatment of aspiration pneumonia, in particular, when the causative bacteria of aspiration pneumonia are bacteria belonging to the genus *Bacteroides,* bacteria belonging to the genus *Prevotella,* bacteria belonging to the genus *Parvimonas,* bacteria belonging to the genus *Veillonella,* or bacteria belonging to the genus *Actinomyces,* 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid exhibits a high therapeutic effect.
Examples of causative bacteria of lung suppuration or lung abscess include bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia,* bacteria belonging to the genus *Fusobacterium,* bacteria belonging to the genus *Bacteroides* and bacteria belonging to the genus *Streptococcus.*
Regarding the treatment of lung suppuration or lung abscess, in particular, when the causative bacteria of lung suppuration or lung abscess are bacteria belonging to the genus *Prevotella,* bacteria belonging to the genus *Porphyromonas,* bacteria belonging to the genus *Fusobacterium,* bacteria belonging to the genus *Leptotrichia,* bacteria belonging to the genus *Peptostreptococcus,* bacteria belonging to the genus *Parvimonas,* bacteria belonging to the genus Veillonella, bacteria belonging to the genus *Tissierella* or the *Streptococcus anginosus* group, 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid exhibits a high therapeutic effect.

Examples of bacteria belonging to the genus *Prevotella* include *P. denticola, P. loescheii, P. melaninogenica, P. intermedia, P. nigrescens, P. pallens, P. buccae, P. oris, P. buccalis, P. oralis, P. bivia, P. disiens, P. pleuritidis, P. bergensis, P. timonensis,* or *P. nanceiencis.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the cases where the causative bacteria of aspiration pneumonia are *P. melaninogenica, P. intermedia,* or *P. buccae* and the cases where the causative bacteria of lung suppuration or lung abscess are *P. melaninogenica, P. intermedia,* or *P. oralis* are more preferably mentioned. Examples of bacteria belonging to the genus *Peptostreptococcus* include *P. anaerobius* and *P. stomatis.*

Examples of bacteria belonging to the genus *Parvimonas* include *P. micra.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the case where the causative bacteria of aspiration pneumonia, lung suppuration or lung abscess are *P. micra* is more preferably mentioned.

Examples of bacteria belonging to the genus *Peptoniphilus* include *Peptoniphilus asaccharolyticus, Peptoniphilus ivorii, Peptoniphilus lacrimalis,* and *Peptoniphilus harei.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the case where the causative bacteria of aspiration pneumonia, lung suppuration or lung abscess are *Peptoniphilus asaccharolyticus* is more preferably mentioned.

Examples of bacteria belonging to the genus *Finegoldia* include *Finegoldia magna.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the case where the causative bacteria of aspiration pneumonia, lung suppuration or lung abscess are *Finegoldia magna* is more preferably mentioned.

Examples of bacteria belonging to the genus *Fusobacterium* include *F. necrophorum, F. nucleatum, F. mortiferum,* and *F. varium.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the cases where the causative bacteria of lung suppuration or lung abscess are *F. nucleatum* or *F. necrophorum* are more preferably mentioned.

Examples of bacteria belonging to the genus *Bacteroides* include *B. fragilis, B. thetaiotaomicron, B. vulgatus, B. ovatus, B. uniformis, B. eggerthii, B. nordii, B. salyersae,* and *B. massiliensis.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the case where the causative bacteria of lung suppuration or lung abscess are *B. fragilis* is more preferably mentioned.

Examples of bacteria belonging to the genus *Porphyromonas* include *P. gingivalis, P. endodontalis, P. asaccharolytica, P. levii,* and *P. uenonis.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the cases where the causative bacteria of lung suppuration or lung abscess are *P. gingivalis* or *P. endodontalis* are more preferably mentioned.

Examples of bacteria belonging to the genus *Leptotrichia* include *L. buccalis, L. hofstadii, L. hongkongensis, L. shahii, L. goodfellowii, L. trevisanii,* and *L. wadei.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the case where the causative bacteria of lung suppuration or lung abscess are *L. buccalis* is more preferably mentioned. Examples of bacteria belonging to the genus *Veillonella* include *V. parvula, V. atypica,* and *V. montpelliensis.*

Examples of bacteria belonging to the genus *Tissierella* include *T. creatinini, T. creatinophila,* and *T. praeacuta.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the case where the causative bacteria of lung suppuration or lung abscess are *T. creatinini* is more preferably mentioned.

Examples of bacteria belonging to the *Streptococcus anginosus* group include *S. intermedius* and S. *constellatus.* From the viewpoint of the therapeutic efficacy of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid, the cases where the causative bacteria of lung suppuration or lung abscess are S. intermedius and S. constellatus are more preferably mentioned.

Examples of bacteria belonging to the genus *Actinomyces* include *A. europaeus, A. georgiae, A. gerencseriae, A. graevenitzii, A. israelii, A. meyeri, A. naeslundii, A. neuii, A. odontolyticus, A. radicidentis, A. radingae, A. turicensis, A. urogenitalis, A. viscocus,* and *Actinomyces* sp. From the viewpoint of the therapeutic efficacy of 7-[(3S,4S)-3-{(cyclopropylamino)methyl}-4-fluoropyrrolldin-1-yl]-6-fluoro-1-(2-fluoroethyl)-8-methoxy-4-oxo-1,4-dihydroquinoline-3-carboxylic acid, the case where the causative bacteria of aspiration pneumonia are A. *odontolyticus* is more preferably mentioned.

A causative bacterium herein is a concept including also bacteria that have acquired drug resistance. Drug

resistance means a phenomenon in which an organism has resistance to a drug and the drug is not effective or becomes less effective. Examples of drug resistance include penicillin resistance, cephalosporin resistance, carbapenem resistance, aminoglycoside resistance, macrolide resistance, lincomycin resistance, trimethoprim-sulfamethoxazole resistance, tetracycline resistance, metronidazole resistance, glycopeptide resistance, oxazolidinone resistance, daptomycin resistance and quinolone resistance.

Examples of pharmaceutically acceptable additives contained along with 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid in the above pharmaceutical composition include excipients, lubricants, binders, disintegrants, stabilizers, flavoring agents, and diluents. These additives are not particularly limited as long as they can be used for the production of pharmaceutical preparations, and, for example, those described in the Pharmaceutical Additives Dictionary "International Pharmaceutical Excipients Council Japan, Yakuji Nippo (2007)" can be used as appropriate.

The therapeutic agent of the present embodiment can be administered to a subject such as a human by applying conventional pharmacologically well-known forms and administration routes. For example, preparations such as powders, tablets, capsules, fine granules, granules, syrups, injections, ophthalmic solutions, aqueous nasal drops, aqueous ear drops and inhalation solutions can be administered orally or parenterally. That is, the therapeutic agent of the present embodiment can be produced by mixing the active ingredient with a physiologically acceptable carrier, excipient, binder, diluent and the like, for example, in the dosage forms as exemplified above.

In the therapeutic agent of the present embodiment, in terms of reducing side effects, making a small-sized preparation that is easy to take, and preventing the appearance of resistant bacteria, minimum daily doses of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof preferably include 10 mg or more, 20 mg or more, 50 mg or more, 100 mg or more, 125 mg or more, and 150 mg or more. Moreover, maximum daily doses preferably include 300 mg or less, 250 mg or less, 200 mg or less, and 175 mg or less. Examples of daily doses of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof include 10 mg or more and 300 mg or less, more preferably 20 mg or more and 250 mg or less, further preferably 50 mg or more and 200 mg or less, further preferably 100 mg or more and 200 mg or less, further preferably 125 mg or more and 175 mg or less, and particularly preferably 150 mg. When a pharmaceutically acceptable salt of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid is used, the value when converted into a free form is used as the above daily dose. The dose for one day may be administered once or may be divided into 2 to 3 times, but an administration once a day is preferable. In addition, if the effect is insufficient, a dose twice the daily dose may be used.

Furthermore, it is preferable to perform loading administration in order to quickly reach the target blood concentration. Loading administration means an administration design for reaching a target blood concentration at an early stage by increasing the daily dose or increasing the number of administrations per day at the initial stage of administration. The initial stage of administration means the first day to the third day of the start of administration, preferably means the first day to the second day of the start of administration, and further preferably the first day of the start of administration. In addition, as an increase in daily dose, preferably twice the daily dose is used.

When performing loading administration, it is preferable to use twice the daily dose on the first day of the start of administration. A more preferable daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of free form.

The dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid hydrochloride is preferably 300 mg on the administration start date and 150 mg on and after the second day of administration. Here, the dose means the value obtained by converting 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid hydrochloride into the free form.

A pharmaceutically acceptable salt can be used as a pharmaceutically acceptable salt of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid. Examples of pharmaceutically acceptable salts include salts of inorganic acids such as hydrochloric acid, hydrobromic acid, sulfuric acid and phosphoric acid, salts of organic acids such as maleic acid, fumaric acid, succinic acid, malic acid, malonic acid, methanesulfonic acid, toluenesulfonic acid, benzenesulfonic acid, lactic acid, oxalic acid, acetic acid, trifluoroacetic acid and tartaric acid, or salts of metals such as sodium, potassium, magnesium, calcium, aluminum, cesium, chromium, cobalt, copper, iron, zinc, platinum and silver. Among these, hydrochloride is particularly preferable.

"Free form" means 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid which is neither a salt, a co-crystal nor a hydrate, and is a

compound with a molecular formula of $C_{21}H_{24}F_3N_3O_4$ and a molecular weight of 439.44.

The therapeutic agent of the present embodiment may be composed solely of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient. Alternatively, the therapeutic agent of the present embodiment may be composed as a pharmaceutical composition containing 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof, and other compounds acting as active ingredients and/or pharmaceutically acceptable additives.

The pharmaceutical composition can contain one or more compounds as other compounds acting as active ingredients and/or as pharmaceutically acceptable additives. The pharmaceutical composition is prepared, for example, by mixing 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof with one or more of other compounds acting as active ingredients and additives.

As described above, according to the present embodiment, a technique relating to a therapeutic agent which has a high therapeutic effect and safety against aspiration pneumonia, lung suppuration or lung abscess, can be provided. By using an appropriate composition as described herein, even when a small dose is used, a sufficient therapeutic effect can be obtained while reducing side effects and reducing the appearance frequency of resistant bacteria.

(Examples)

Hereinafter, the present invention will be further described in detail by showing Examples, but the scope of the present invention is not limited by these Examples. According to the method disclosed in International Publication No. WO 2016/195014, a 150 mg injection of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid (hereinafter also referred to as investigational new drug A) was prepared.

"150 mg" in the 150 mg injection indicates the weight when 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid hydrochloride is converted into the free form. When preparing the injection, 162.5 mg (converted to free form: 150 mg) of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid hydrochloride is used.

(Test Example 1) Aspiration pneumonia

The investigational new drug A was administered intravenously for 7 to 14 days to 13 subjects suspected of having aspiration pneumonia meeting the following criteria.

- A clear infiltrative shadow appearing acutely on chest X-rays or CT images taken within 48 hours before the start of administration at the age of 16 years or older, is observed.
- Confirmation of clear aspiration, choking or dysphagia, has a disease or history of disease with potential dysphagia.
- The patient presents with symptoms and inflammation that are characteristic of aspiration pneumonia.
  On the first day of the start of administration, two doses of investigational new drug A (300 mg/day) were used, and on the second day of administration, one dose of investigational new drug A (150 mg/day) was used, and then the same dose (150 mg/day) was maintained. The administration of the injection was performed intravenously over about 1 hour per dose.

(Test Example 2) Lung suppuration or lung abscess

The investigational new drug A was administered intravenously for 7 to 14 days to 11 subjects suspected of having lung suppuration or lung abscess meeting the following criteria.

- On chest X-rays or CT images taken within 48 hours before the start of administration at the age of 16 years or older, a massive shadow or a shadow with cavities inside (nodular shadow, mass shadow) is observed. Regardless of the presence of a niveau due to pus accumulation.

- The patient presents with symptoms and inflammation that are characteristic of lung suppuration or lung abscess.

On the first day of the start of administration, two doses of investigational new drug A (300 mg/day) were used, and on the second day of administration, one dose of investigational new drug A (150 mg/day) was used, and then the

same dose (150 mg/day) was maintained. The administration of the injection was performed intravenously over about 1 hour per dose.

The clinical efficacy of Test Examples 1 and 2 were determined by setting the following criteria, based on the criteria for clinical efficacy of pneumonia described in Clinical evaluation method of new antibacterial drugs in respiratory infections (2nd edition), Japanese Journal of Chemotherapy. 2012; 60(1): 30-45. 9). The primary endpoint was the efficacy rate at the end of administration or discontinuation of investigational new drug A.

The end of administration herein means the evaluation date on the day after the administration of the investigational new drug A is completed. Moreover, the time of discontinuation means the date on which the evaluation was performed within 3 days from the last administration date or the discontinuation judgment date of the investigational new drug A. Furthermore, "the end of administration or the time of discontinuation" is expressed as End of Treatment (EOT). In addition, CRP is an abbreviation for C-reactive protein, and is one of the acute phase reactants produced in a short time in response to various inflammations. It is a useful index for the observation of therapeutic effects since it increases in a few hours in bacterial infections such as pneumonia and decreases rapidly as the inflammation subsides.

• Early drug efficacy evaluation and End of Treatment (EOT)

The early drug efficacy evaluation was determined according to Table 1, 3 days after administration, in three stages: "Early therapeutic effect", "No early therapeutic effect", and "Undeterminable". "Early therapeutic effect" was defined as a case in which significant improvement is observed 3 days after administration (regardless of whether administration was completed or continued after 4 days). In addition, in cases where CRP values and chest X-rays did not improve 3 days after administration compared to before the start of administration, even though CRP or chest X-ray findings were unchanged or worsened, if clinical symptoms and body temperature had improved, it was judged as "Early therapeutic effect".

If CRP or chest X-ray findings did not change or worsen, and clinical symptoms and body temperature did not change or improve, it was determined as "No early therapeutic effect", and fully considering the safety of the subject, the investigator or the like made an appropriate decision, such as discontinuing the clinical trial and switching to the administration of other antibacterial drugs. Samples for microbiological evaluation were collected before starting an appropriate alternative antibacterial treatment.

In addition, if the discontinuation date is the start date of administration (day 0) or the second day of administration (day 1), the determination of early drug efficacy was regarded as unnecessary. In the case of the third day of administration (day 2) or later, it was determined using the test results at the time of discontinuation.
At the end of treatment (EOT), the clinical efficacy at the end of administration or at the time of discontinuation were determined according to Table 1 in three stages: "Effective", "Ineffective", and "Undeterminable". When the treatment was discontinued after the next day after the end of the administration, the clinical efficacy at the end of the administration was determined, and the determination of the clinical efficacy at the time of discontinuation was not required. In addition, it was judged as "Ineffective" when the treatment was discontinued or when it was changed to an alternative antibacterial treatment after the end of administration of the investigational new drug. However, this does not apply to cases where it was judged "Effective" according to the criteria in Table 1 at the end of treatment (EOT), even if it was changed to an alternative antibacterial treatment. When changing to an alternative antibacterial treatment, in principle, prescribed tests, examinations and judgments at the end of treatment were made before the change.

[Table 1]

Table 1. Early drug efficacy evaluation and end of treatment criteria

| | |
|---|---|
| Early therapeutic effect/ Effective | When the following a. is satisfied and either b. or c. is satisfied, and the remaining items have not worsened<br>a. Disappearance or improvement of symptoms/findings of the primary disease<br>• Determined by the fever, cough, sputum (amount, aspect), dyspnea, chest pain and wet rales.<br>• One or more symptoms/findings has improved.<br>• In cases having a fever at the start of the study (time of inclusion), an improvement of the fever is required.<br>• If it has decreased from the body temperature at the start of the study (time of inclusion), it is regarded as an improvement of the fever even at 37°C or more. |

(continued)

| | |
|---|---|
| | b. All abnormal findings in chest images have disappeared or improved from the worst state<br>• Determined based on shadow spread and density.<br>c. Inflammatory findings have disappeared or improved<br>• Either an improvement to a white blood cell count of 9,000/mm3 or less or a decrease from the maximum CRP value is met, and there is no item that has worsened. Note that it is not determined as "worsened" if the white blood cell count is within the normal range at the clinical laboratory. |
| No early therapeutic effect/ Ineffective | When the above conditions for "Early therapeutic effect" or "Effective" are not satisfied |
| Undeterminable | When one of the following criteria is met<br>a. When information on symptoms/findings is lacking, such as when there is no visit at the end of administration<br>b. When there is a clear reason other than the primary disease for the cause of the worsening of body temperature, white blood cells and CRP |

Tables 2 and 3 show the results of the early drug efficacy evaluation and the end of treatment in Test Examples 1 and 2.

[Table 2]

Table 2. Early drug efficacy evaluation

| | Clinical efficacy | | | Efficacy rate |
|---|---|---|---|---|
| | Effective | Ineffective | Undeterminable | |
| Test Example 1 | 12 | 1 | 0 | 92% |
| Test Example 2 | 9 | 2 | 0 | 82% |

[Table 3]

Table 3. End of Treatment

| | Clinical efficacy | | | Efficacy rate |
|---|---|---|---|---|
| | Effective | Ineffective | Undeterminable | |
| Test Example 1 | 12 | 0 | 1 | 100% |
| Test Example 2 | 10 | 1 | 0 | 91% |

The efficacy rate is the value obtained by the following formula.

Efficacy rate = (number of subjects rated as "Effective" ÷ number of subjects rated as "Effective" or "Ineffective") × 100 (%)

Table 2 and Table 3 show that 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof has

a high therapeutic effect on aspiration pneumonia, lung suppuration or lung abscess. In particular, the efficacy rate at the end of treatment was remarkably high, with 100% for aspiration pneumonia and 91% for lung suppuration or lung abscess.

Table 4 and Table 5 show the microbiological efficacy of Test Examples 1 and 2 according to the causative bacteria.

[Table 4]

Table 4. Microbiological efficacy according to the causative bacteria (Aspiration pneumonia)

| Causative bacteria | | Case number | Result | |
|---|---|---|---|---|
| | | | Eradicated | Persisted |
| Anaerobe | | 19 | 17 | 2 |
| genus Actinomyces | *A. odontolyticus* | 1 | 1 | 0 |
| genus Parvimonas | *P. micra* | 1 | 1 | 0 |
| genus Veillonella | *Veillonella sp.* | 7 | 5 | 2 |
| genus Bacteroides | *Bacteroides sp.* | 2 | 2 | 0 |
| genus Prevotella | *Prevotella sp.* | 6 | 6 | 0 |
| | *P. buccae* | 1 | 1 | 0 |
| | *P. intermedia* | 1 | 1 | 0 |

[Table 5]

Table 5. Microbiological efficacy according to the causative bacteria (Lung suppuration/lung abscess)

| Causative bacteria | | Case number | Result | |
|---|---|---|---|---|
| | | | Eradicated | Persisted |
| Anaerobe | | 16 | 15 | 1 |
| genus Parvimonas | *P. micra* | 2 | 2 | 0 |
| genus Veillonella | *Veillonella sp.* | 2 | 2 | 0 |
| genus Bacteroide | *B.fragilis* | 1 | 1 | 0 |
| genus Fusobacterium | *F. necrophorum* | 1 | 1 | 0 |
| | *F. nucleatum* | 4 | 4 | 0 |
| genus Leptotrichia | *Leptotrichia buccalis* | 1 | 1 | 0 |
| genus Porphyromonas | *P. gingivalis* | 1 | 1 | 0 |
| genus Prevotella | *Prevotella sp.* | 1 | 1 | 0 |
| | *P. intermedia* | 1 | 0 | 1 |
| | *P. melaninogenica* | 1 | 1 | 0 |
| | *P. oralis* | 1 | 1 | 0 |

Table 4 and Table 5 show that 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof has a high antibacterial effect on the causative bacteria of aspiration pneumonia, lung suppuration or lung abscess.

Industrial Applicability

According to the present embodiment, it is possible to provide a therapeutic agent for aspiration pneumonia, lung suppuration or lung abscess, which is industrially useful.

## Claims

1. A therapeutic agent for aspiration pneumonia, lung suppuration or lung abscess comprising 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient.

2. A therapeutic agent for aspiration pneumonia comprising 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient.

3. A therapeutic agent for lung suppuration or lung abscess comprising 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof as an active ingredient.

4. The therapeutic agent according to claim 1, wherein causative bacteria of the aspiration pneumonia, lung suppuration or lung abscess are one or more bacteria selected from the group consisting of bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia* and bacteria belonging to the genus *Fusobacterium.*

5. The therapeutic agent according to claim 2, wherein causative bacteria of the aspiration pneumonia are one or more bacteria selected from the group consisting of bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia* and bacteria belonging to the genus *Fusobacterium.*

6. The therapeutic agent according to claim 3, wherein causative bacteria of the lung suppuration or lung abscess are one or more bacteria selected from the group consisting of bacteria belonging to the genus *Prevotella,* bacteria belonging to the genus *Peptostreptococcus,* bacteria belonging to the genus *Parvimonas,* bacteria belonging to the genus *Peptoniphilus,* bacteria belonging to the genus *Finegoldia* and bacteria belonging to the genus *Fusobacterium.*

7. The therapeutic agent according to claim 1, wherein causative bacteria of the aspiration pneumonia, lung suppuration or lung abscess are one or more bacteria selected from the group consisting of bacteria belonging to the genus *Bacteroides,* bacteria belonging to the genus *Prevotella,* bacteria belonging to the genus *Porphyromonas,* bacteria belonging to the genus *Fusobacterium,* bacteria belonging to the genus *Leptotrichia,* bacteria belonging to the genus *Peptostreptococcus,* bacteria belonging to the genus *Parvimonas,* bacteria belonging to the genus *Veillonella,* bacteria belonging to the genus *Tissierella, Streptococcus anginosus* group, and bacteria belonging to the genus *Actinomyces.*

8. The therapeutic agent according to claim 2, wherein causative bacteria of the aspiration pneumonia are one or more bacteria selected from the group consisting of bacteria belonging to the genus *Bacteroides,* bacteria belonging to the genus *Prevotella,* bacteria belonging to the genus *Parvimonas,* bacteria belonging to the genus *Veillonella* and bacteria belonging to the genus *Actinomyces.*

9. The therapeutic agent according to claim 3, wherein causative bacteria of the lung suppuration or lung abscess are one or more bacteria selected from the group consisting of bacteria belonging to the genus *Bacteroides,* bacteria belonging to the genus *Prevotella,* bacteria belonging to the genus *Porphyromonas,* bacteria belonging to the genus *Fusobacterium,* bacteria belonging to the genus *Leptotrichia,* bacteria belonging to the genus *Peptostreptococcus,* bacteria belonging to the genus *Parvimonas,* bacteria belonging to the genus *Veillonella,* bacteria belonging to the genus *Tissierella,* and *Streptococcus anginosus* group.

10. The therapeutic agent according to claim 1, wherein a daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid.

**11.** The therapeutic agent according to claim 2, wherein a daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid.

**12.** The therapeutic agent according to claim 3, wherein a daily dose of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid or a pharmaceutically acceptable salt thereof is 300 mg on the administration start date and 150 mg on and after the second day of administration, in terms of 7-[(3S,4S)-3-{(cyclopropylamino)methyl}-4-fluoropyrrolidin-1-yl]-6-fluoro-1-(2-fluoroethyl)-8-methoxy-4-oxo-1,4-dihydroquinoline-3-carboxylic acid.

**INTERNATIONAL SEARCH REPORT**

International application No.
PCT/JP2018/022846

A. CLASSIFICATION OF SUBJECT MATTER
Int.Cl. A61K31/4709(2006.01)i, A61P11/00(2006.01)i, A61P31/04(2006.01)i

According to International Patent Classification (IPC) or to both national classification and IPC

B. FIELDS SEARCHED

Minimum documentation searched (classification system followed by classification symbols)
Int.Cl. A61K31/4709, A61P11/00, A61P31/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY/MEDLINE/EMBASE/BIOSIS (STN)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2016/148066 A1 (KYORIN PHARMACEUTICAL CO., LTD.) 22 September 2016, test examples, reference examples, paragraph [0021] & US 2018/0042918 A1, test examples, reference example 4, paragraphs [0075], [0076] & EP 3269369 A1 & KR 10-2017-0123695 A & CN 107427507 A | 1-3, 10-12<br>4-9 |
| Y<br>A | ALLEWELT, M. et al., "Ampicillin + sulbactam vs. clindamycin ± cephalosporin for the treatment of aspiration pneumonia and primary lung abscess", Clin. Microbiol. Infect., 2004, vol. 10, pp. 163-170, ISSN: 1198-743X, in particular, table 3, abstract | 1-3, 10-12<br>4-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 September 2018 (05.09.2018) | 18 September 2018 (18.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/022846

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | EL-SOLH, Ali A. et al., "Microbiology of Severe Aspiration Pneumonia in Institutionalized Elderly", Am. J. Respir. Crit. Care. Med., 2003, vol. 167, pp. 1650-1654, ISSN: 1073-449X, table 3, abstract | 1-3, 10-12<br>4-9 |
| Y<br>A | BARTLETT, John G. et al., "The Bacteriology of Aspiration Pneumonia", The American Journal of Medicine, 1974, vol. 56, pp. 202-207, ISSN: 0002-9343, table III, abstract | 1-3, 10-12<br>4-9 |
| Y<br>A | WO 2016/195014 A1 (KYORIN PHARMACEUTICAL CO., LTD.) 08 December 2016, examples 16, 17 & US 2018/0153875 A1, examples 16, 17 & EP 3305294 A1 | 10-12<br>4-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005026147 A
- WO 2013069297 A
- WO 2016195014 A

### Non-patent literature cited in the description


- *The Journal of the Japanese Society of Internal Medicine,* 10 November 2010, vol. 99 (11), 2746-2751
- *The Japanese Respiratory Society, Medical/Care-related Pneumonia Clinical Practice Guidelines,* 23
- *Infection,* 2008, vol. 36 (1), 23-30
- *Expert Review of Respiratory Medicine,* 2007, vol. 1 (1), 111-119
- *The Japanese Respiratory Society, Adult Pneumonia Clinical Practice Guidelines,* 2017, 24
- *Annals of The Japanese Respiratory Society,* 2011, vol. 49 (9), 623-628
- *Nippon Kagaku Ryoho Gakkai Zasshi,* 1999, vol. 47 (1), 196-203
- International Pharmaceutical Excipients Council Japan, Yakuji Nippo (2007). Pharmaceutical Additives Dictionary. 2007
- Japanese Journal of Chemotherapy. 2012, vol. 60, 30-45